# EUROPÄISCHE PATENTSCHRIFT

(11) **EP 3 851 019 B1**
(45) Veröffentlichungstag und Bekanntmachung des Hinweises auf die Patenterteilung: **06.09.2023**
(21) Anmeldenummer: 21151700.8
(22) Anmeldetag: 14.01.2021
(51) Int. Cl.: A61B 1/00, A61M 39/22, F16K 5/02, F16K 27/06, A61B 1/015

(54) **ABSPERRHAHN FÜR EIN ENDOSKOP**
PLUG VALVE FOR AN ENDOSCOPE
ROBINET D'ARRÊT POUR UN ENDOSCOPE

(30) Priorität: 20.01.2020 DE 102020101206
(43) Veröffentlichungstag der Anmeldung: 21.07.2021
(73) Patentinhaber: Olympus Winter & Ibe GmbH, 22045 Hamburg (DE)
(72) Erfinder: Miersch, Hannes, 22149 Hamburg (DE); Schwendele, Andrea, 22767 Hamburg (DE)
(74) Vertreter: Hoener, Matthias

(56) Entgegenhaltungen:
- DE-A1- 4 226 770
- DE-A1-102014 002 158
- DE-A1-102019 203 684
- US-A- 6 012 702

## Beschreibung

Die Erfindung betrifft Absperrhähne für einen Flüssigkeitskanal eines medizinischen Endoskops der im Anspruch 1 genannten Art sowie medizinische Endoskope der im Anspruch 10 genannten Art.

Medizinische Endoskope sind Instrumente mit einem länglichen, rohrförmigen, flexiblen oder starren Schaft und einer Optik, die zur optischen, minimal invasiven Untersuchung von Innenräumen des menschlichen Körpers und für minimal invasive Eingriffe verwendet werden. Durch den Schaftabschnitt der Endoskope können Durchgangsinstrumente, wie Steinfangkörbchen, Instrumente zur elektrochirurgischen Resektion oder Zangen, zur Eingriffsstelle geführt werden. Um während des Eingriffs entstehende lokale Blutungen wegzuspülen und das Gewebe zum Beispiel vor Hitzeschäden durch eine hochfrequente elektrochirurgische Anwendung zu schützen, sind die Endoskope häufig mit einer Spüleinrichtung versehen, die das vor dem distalen Schaftende liegende Gewebe dauerhaft umspült. Die Spülflüssigkeit kann durch entsprechende separate Flüssigkeitskanäle, durch das Innenrohr oder durch das Hüllrohr geleitet werden. Um den Zu- und Abstrom der Flüssigkeit kontrollieren zu können, weisen die Flüssigkeitskanäle in der Regel in ihrem proximalen Endbereich einen Absperrhahn auf. Entsprechende Absperrhähne sind beispielsweise in DE 101 26 540 A1 und DE 10 2014 002 158 B4 beschrieben.

Die Absperrhähne bestehen üblicherweise aus einem Gehäuse und einem darin drehbaren Küken, deren Wände dichtend aneinander angrenzen. Die großflächige Anlagefläche zwischen Gehäuse und Küken ist im montierten Zustand schwer zu reinigen und zu sterilisieren, da der Abstand zwischen den beiden Teilen zu klein ist, um während des Autoklavierens sicher vom Dampf erreicht werden zu können. Daher muss bislang das Küken über eine Schraube gelöst oder ausgerastet und aus dem Gehäuse entnommen werden. Hierdurch wird der kritische Spalt zwischen den beiden Teilen geöffnet, sodass der Dampf die Flächen während des Autoklavierens ungehindert erreichen kann. Nach dem Autoklavieren muss das Instrument dann allerdings wieder zusammengesetzt werden, wodurch sich die Vorbereitungszeit vor der OP verlängert. Einige Instrumente umfassen bis zu vier verschiedene Absperrhähne, deren Küken nach einer solchen Demontage und Reinigung zunächst wieder den richtigen Flüssigkeitskanälen zugeordnet werden müssen. Es kann daher einige Zeit dauern, bis alle Absperrhähne eines Endoskops nach der Reinigung wieder zusammengesetzt und einsatzbereit sind. Dazu kommt, dass die Küken oft relativ klein sind, sodass die Gefahr besteht, sie während eines Autoklaviervorgangs - insbesondere beim gleichzeitigen Autoklavieren mehrerer Endoskope - zu verlieren.

In der US 6,012,702 wird zur Sicherstellung der Sterilisierbarkeit aller Flächen und Verhinderung eines Verlusts des Kükens während der Reinigung eine zweistufige flexible Verrastung des Kükens in dem Hahngehäuse vorgeschlagen. Dazu weist das Küken zwei parallele, konzentrisch um seine Längsachse verlaufende Ringnuten auf. Das Küken ist in einer Arbeitsposition mit dem Gehäuse form- und kraftschlüssig verrastet. In dieser Arbeitsposition liegen Dichtflächen des Kükens dichtend an Dichtflächen des Gehäuses an und eine Ringfeder liegt in der ersten Ringnut. Unter Überwindung der Verrastung ist das Küken in eine Zwischenposition bewegbar, in der sich die Dichtflächen außer Anlage befinden und die Ringfeder in der zweiten Nut liegt. In der Zwischenposition kann Sterilisationsgas durch den Spalt zwischen den Dichtflächen strömen, sodass eine fehlerhafte Sterilisation ausgeschlossen wird.

Nachteilig an dieser Ausführungsweise ist jedoch, dass die Rastgeometrien für die Arbeitsposition und die Zwischenposition gleich ausgeführt sind und die beiden Position nur schwer zu unterscheiden sind. Hierdurch wird das Risiko erhöht, dass in der Arbeitsposition sterilisiert oder in der Zwischenposition gearbeitet wird.

Es besteht daher ein Bedarf an Absperrhähnen für Endoskope, die leicht und fehlerfrei gereinigt werden können, für deren Küken keine Verlust- oder Verwechslungsgefahr bei der anschließenden Montage besteht und in denen Arbeits- und Sterilisationsposition leicht zu unterscheiden sind.

### Beschreibung

Gelöst wird diese Aufgabe durch einen Absperrhahn mit den Merkmalen von Anspruch 1 und ein Endoskop mit den Merkmalen von Anspruch 10. Erfindungsgemäß ist insbesondere vorgesehen, in der Konusfläche des Kükens eine Quernut auszubilden, die einen geraden Nutboden aufweist.

In einem ersten Aspekt betrifft die Erfindung einen Absperrhahn für einen Flüssigkeitskanal eines medizinischen Endoskops, mit einem Gehäuse, einem Federelement und einem drehbar in dem Gehäuse gelagerten Küken, wobei das Küken in einer Arbeitsstellung mit einer Konusfläche an einer Dichtfläche des Gehäuses anliegt und in eine Mediumszugangsstellung bringbar ist, in der sich die Konusfläche und die Dichtfläche außer Anlage befinden und das Gehäuse und das Küken verbunden bleiben, dadurch gekennzeichnet, dass das Küken in der Außenwand eines Konusteils eine oder mehrere quer zur Längsachse des Konusteils verlaufende Quernuten aufweist, deren Nutboden gerade sind, und das Federelement in der Mediumszugangsstellung in die eine oder mehreren Quernuten eingreift.

Der erfindungsgemäße Absperrhahn ist geeignet, sicherzustellen, dass der Absperrhahn während der Sterilisation in der Mediumszugangsstellung und geöffnet verbleibt, sodass entlang des Spaltes zwischen dem Kükenkonus und dem Gehäuse Sterilisationsgas eindringen kann. Die Mediumszugangsstellung ist für das medizinische Fachpersonal und das Reinigungspersonal einfach daran zu erkennen, dass sich der Hahn in dieser Stellung nicht oder nur mit großen Schwierigkeiten schließen lässt.

Der erfindungsgemäße Absperrhahn ist ferner geeignet, den Flüssigkeitsstrom in einem Flüssigkeitskanal zu regulieren. Durch Drehen des Kükens des Absperrhahns um seine Drehachse kann der Flüssigkeitsstrom durch den Flüssigkeitskanal reduziert und vollständig gestoppt werden (Geschlossenstellung). Zu diesem Zweck durchläuft der Flüssigkeitskanal den Absperrhahn, d.h. der Absperrhahn weist in seinem Inneren einen Flüssigkeitskanal auf, dessen Durchmesser dem des betreffenden Kanals im Endoskop im Wesentlichen gleicht. Als Offenstellung des Absperrhahns wird erfindungsgemäß die Stellung angesehen, in welcher der Hahn vollständig geöffnet ist, d.h. in welcher die maximale Flüssigkeitsmenge durch den Hahn fließen kann.

Der Absperrhahn weist ein Gehäuse (Hahngehäuse), ein Federelement und ein Küken auf oder besteht aus diesen drei Elementen. Das Küken ist mit dem Gehäuse und dem Federelement in zwei Stellungen verrastbar, in einer Arbeitsstellung und einer Mediumszugangsstellung (auch: Reinigungsstellung oder Sterilisationsstellung). Der Begriff "Medium" bezeichnet in diesem Zusammenhang insbesondere Sterilisations- und Reinigungsflüssigkeiten und -gase. Der Begriff "Zugang" bedeutet, dass diese Flüssigkeiten und Gase in dieser Stellung zwischen das Küken und das Gehäuse eintreten können, insbesondere zwischen Dicht- und Konusflächen. Mit anderen Worten ist das Küken in eine Arbeitsstellung und eine Mediumszugangsstellung bringbar. In beiden Stellungen sind Küken, Gehäuse und Federelement verrastet miteinander verbunden. Ein Auseinanderfallen des Absperrhahns während der Reinigung wird so verhindert.

In der Arbeitsstellung liegt das Küken mit einer Konusfläche an einer Dichtfläche des Gehäuses an. Die Konusfläche liegt dabei dichtend, d.h. vorzugsweise flüssigkeitsdicht, stärker bevorzugt auch im Wesentlichen gasdicht, an der Dichtfläche des Gehäuses an. Dabei kann vorzugsweise keine Flüssigkeit und/oder Gas aus dem Flüssigkeitskanal zwischen Küken und Gehäuse hindurch in das Hahnäußere austreten. Dicht- und Konusfläche liegen daher vorzugsweise entlang des gesamten Umfangs des Konusteils aneinander an und sowohl auf der Griffseite als auch auf der dem Griff abgewandten Seite des Flüssigkeitskanals. In der Arbeitsstellung sind das Küken und das Gehäuse rastend miteinander verbunden. Die Arbeitsstellung kann auch erste Raststellung bezeichnet werden.

In der Mediumszugangsstellung befinden sich die Konusfläche und die Dichtfläche außer Anlage. Ein Flüssigkeits- und Gaseintritt zwischen der Konusfläche und der Dichtfläche ist somit möglich und erwünscht. Dabei kann vorzugsweise Flüssigkeit und/oder Gas aus dem Flüssigkeitskanal zwischen Küken und Gehäuse hindurch in das Hahnäußere austreten. Der zwischen Konusfläche und Dichtfläche in der Mediumszugangsstellung bestehende Spalt ist somit ausreichend groß, um die Sterilisierbarkeit der beiden Flächen sicherzustellen. Wie oben beschrieben, bleiben das Gehäuse und das Küken dennoch rastend miteinander verbunden. Die Mediumszugangsstellung kann auch als zweite Raststellung bezeichnet werden.

Prinzipiell kann der Absperrhahn sowohl in der Arbeitsstellung als auch in der Mediumszugangsstellung in die Offenstellung (Flüssigkeitskanal offen) und in die Geschlossenstellung gebracht werden. Dies erfolgt in der Regel durch Drehung des Kükens um die Längsachse seines Konusteils, zum Beispiel unter Verwendung eines Griffteils. Um eine besonders gute Reinigungswirkung zu erzielen, ist es jedoch bevorzugt, dass sich der Absperrhahn während der Reinigung in der Offenstellung befindet.

Das Küken weist, in Richtung seiner Drehachse hintereinanderliegend, ein Konusteil und ein Griffteil auf. Das Küken ist drehbar in dem Gehäuse gelagert. Das Konusteil des Kükens ist kegelstumpfförmig und wird von einem Flüssigkeitskanal durchspannt, dessen Längsachse quer zur Drehachse des Kükens verläuft. Als Außenwand des Konusteils wird hierin seine Mantelfläche angesehen. Die dem Griffteil abgewandte Stirnfläche des Konusteils, wird hierin nicht als Teil der Außenwand angesehen. Die Außenwand des Konusteils umfasst somit die Konusfläche, die dichtend an die Dichtfläche des Gehäuses anlegbar ist. Die Konusfläche stellt somit mindestens einen Teil der Mantelfläche des Konusteils dar.

Das Küken weist in der Außenwand des Konusteils eine oder mehrere quer zur Längsachse des Konusteils verlaufende Quernuten auf, deren Nutboden gerade sind. Die Quernuten sind geeignet, in einer Verrastungsstellung (Mediumszugangsstellung) einen Abschnitt des Federelements aufzunehmen. Sie sind entsprechend zumindest teilweise größenkomplementär zu dem Abschnitt ausgebildet. Die Quernut kann einen eckigen, teilkreisförmigen, U-förmigen oder trapezförmigen Querschnitt aufweisen, wobei ein im Wesentlichen trapezförmiger Querschnitt bevorzugt ist, d.h. ein Querschnitt mit nach außen geschrägter Wand.

Das Küken kann beispielsweise eine, zwei, drei oder vier Quernuten aufweisen, wobei zwei Quernuten bevorzugt sind. Weist das Küken mehr als eine Quernut auf, so sind diese vorzugsweise paarweise derart angeordnet, dass sich jeweils die zwei Quernuten eines Paares auf dem Konusteil gegenüber liegen. In einer Ausführungsform weist das Küken somit zwei Quernuten auf, die sich vorzugsweise auf dem Konusteil gegenüber liegen.

Die Quernuten verlaufen jeweils quer zur Längsachse des Konusteils. Dies bedeutet, dass die Längsachse der Quernut quer zur Längsachse des Konusteils angeordnet ist.

Darüber hinaus sind die erfindungsgemäßen Quernuten vorzugsweise derart an dem Küken angeordnet, dass das an anderer Stelle hierin beschriebene Federelement, bzw. ein Abschnitt des Federelements, in ihnen liegen kann, wenn sich der Hahn in einer Offenstellung befindet. In der Offenstellung ist entsprechend die Längsachse der Quernut parallel zu der Längsachse des Abschnitts des Federelements.

Dadurch stellen die Quernuten sicher, dass der Hahn während der Sterilisation des Instruments in der Offenstellung verbleibt. Vorzugsweise ist der Absperrhahn in der Mediumszugangsstellung in der Offenstellung, und vorzugsweise in der Mediumszugangsstellung nicht in die Geschlossenstellung bringbar, oder nur unter Aufwendung einer Kraft, die größer ist als die zur Drehung des Absperrhahns in einer vollständig konzentrischen Nut, zum Beispiel der ersten Ringnut. Die Kraft, die notwendig ist, um das Küken in der Mediumszugangsstellung in die Geschlossenstellung zu bringen, ist vorzugsweise mindestens doppelt so groß, wie die Kraft, die zur Drehung des Kükens in der Arbeitsstellung in die Geschlossenstellung notwendig ist.

Dies wird durch die gerade Ausbildung des Nutbodens der Quernut erreicht. Im Gegensatz zu den konzentrisch verlaufenden Ringnuten, verläuft die Quernut nicht rund um das Konusteil herum sondern gerade und einseitig am Konusteil. Hierdurch entsteht an den Nutenden ein Vorsprung in der Mantelfläche des Konusteils, der von dem Federelement bei einer Drehung des Kükens unter Aufwendung einer erhöhten Kraft zu überwinden wäre. Entsprechend des üblichen Verständnisses des Begriffs bezeichnet das Merkmal "Nutboden" die Innenseite der Nut, die ihrer offenen Seite - d.h. der Seite, die für den Eingriff des Federelements vorgesehen ist - gegenüberliegt. Hiervon zu unterscheiden sind die seitlich von dem Nutboden angeordneten Innenseiten, welche die Verbindung zwischen der offenen Seite und dem Nutboden darstellen.

Das Küken wird in der Regel parallel zur Quernut eine erste konzentrisch verlaufende Ringnut aufweisen. In diese Ringnut greift in der Arbeitsstellung das Federelement ein. Auf diese Weise wird das Küken in der Arbeitsstellung in dem Gehäuse verrastet. Die Ringnut bildet somit eine kreisförmig um die Längsachse des Konusteils verlaufende Aussparung in der Mantelfläche des Konusteils. Vorzugsweise ist die Ringnut regelmäßig ausgebildet, d.h. der Querschnitt der Ringnut ist über im Wesentlichen die gesamte Länge der Ringnut identisch in Größe und Form oder im Wesentlichen identisch. Die erste Ringnut kann Querschnittsformen aufweisen, wie oben für die Quernut angegeben, wobei für die erste Ringnut eine im Wesentlichen trichterförmige Querschnittsform bevorzugt ist.

Die erste Ringnut ist von der Quernut durch eine Ringschulter am Küken getrennt. Die erste Ringnut ist vorzugsweise zwischen dem Griffteil des Kükens und der Quernut am Konusteil angeordnet. Eine umgekehrte Anordnung, in der die Quernut zwischen der ersten Ringnut und dem Griffteil angeordnet ist, ist aber ebenfalls denkbar. In der ersteren Ausführungsform ist die Quernut in einem Teil des Konusteils angeordnet, der einen kleineren Durchmesser aufweist, als der Teil in dem die Ringnut angeordnet ist. In der letzteren Ausführungsform ist die Quernut in einem Teil des Konusteils angeordnet, der einen größeren Durchmesser aufweist, als der Teil in dem die erste Ringnut angeordnet ist.

Die eine oder mehrere Quernuten können jeweils als Abschnitt einer zweiten Ringnut ausgebildet sein, wobei die zweite Ringnut außerhalb der einen oder mehreren Quernuten parallel zu der ersten Ringnut konzentrisch verläuft. Die zweite Ringnut verhindert ein Herausfallen des Kükens, wenn das Küken aus der Mediumszugangsstellung in die Arbeitsstellung gedreht wird. Die zweite Ringnut kann in den Bereichen, in denen sie nicht von den einen oder mehreren Quernuten unterbrochen wird, in gleicher Weise ausgebildet sein, wie oben für die erste Ringnut geschildert ist.

Alternativ oder zusätzlich kann das Küken in der Außenwand seines Konusteils eine oder mehrere Verbindungsnuten aufweisen, die jeweils ein Ende einer Quernut mit der ersten Ringnut verbinden. Die eine oder mehrere Verbindungsnuten sind gewindeartig ausgebildet und bewirken, dass das Küken automatisch in der Offenstellung verrastet, wenn es in die Mediumszugangsstellung bewegt wird. Die Verbindungsnuten können entsprechend auch als Gewindenuten bezeichnet werden. Vorzugsweise ist die Zahl der Verbindungsnuten gleich der Zahl der Quernuten.

Die eine oder mehreren Verbindungsnuten sind schräg zur Längsachse der ersten Ringnut angeordnet. Sie verlaufen vorzugsweise ausgehend von dem in Drehrichtung des Kükens liegenden Ende einer Quernut in Drehrichtung schräg in Richtung der ersten Ringnut. Hierbei bezeichnet die 'Drehrichtung' die Richtung in der das Küken um seine Längsachse gedreht werden muss, um den Hahn von der Offenstellung in die Geschlossenstellung zu drehen.

Das Gehäuse des Kükens kann mit einem Schaftabschnitt des Endoskops einteilig ausgebildet sein oder als separates Teil in das Endoskop eingefügt werden. Das Gehäuse weist in der Regel eine übliche Quaderform auf, die den Konusteil des Kükens in ihrem Inneren aufnehmen kann. Zu diesem Zweck weist das Gehäuse ferner einen inneren konusförmigen Hohlraum auf, der zum Einführen des Kükens wenigstens einseitig offen ist. Der Hohlraum ist im Wesentlichen Durchmesser-komplementär zum Konusteil des Kükens, insbesondere komplementär zu dem Durchmesser des Kükens, gemessen an seinen dichtenden Konusflächen. Nach Einführen des Kükens in das Gehäuse grenzen die Konusflächen des Kükens daher an die Innenwände des Hohlraums an, wenn sich der Hahn in seiner Arbeitsstellung befindet, d.h. das Federelement in die erste Ringnut eingreift. Die dichtenden, in der Arbeitsstellung an die Konusflächen des Kükens angrenzenden Abschnitte der Innenwände werden daher hierin als Dichtflächen des Gehäuses bezeichnet. In der Mediumszugangsstellung, d.h. in der Stellung in der das Federelement in die Quernut eingreift, besteht dagegen zwischen den Dichtflächen und den Konusflächen ein Spalt.

Der Hohlraum des Gehäuses weist ferner mindestens eine Ringschulter auf, die konzentrisch um die Längsachse des Hohlraums bzw. des Konusteils des Kükens verläuft. Die Ringschulter greift in der Arbeitsstellung des Hahns in die Quernut ein. Sie kann daher form- und größenkomplementär zu der Quernut bzw. der Verbindung aus Quernut und zweiter Ringnut ausgebildet sein. In der Mediumszugangsstellung des Hahns kann die Ringschulter beispielsweise an der Stirnfläche des Konusteils angeordnet sein.

Der erfindungsgemäße Hahn umfasst ferner ein Federelement, das zwischen dem Gehäuse und dem Küken angeordnet ist. Das Federelement ist geeignet, das Küken innerhalb des Gehäuses zu verrasten. Dazu übt das Federelement eine Federkraft in Richtung der Längsachse des Konusteils aus. In der Arbeitsstellung und der Mediumszugangsstellung greift das Federelement jeweils in die erste Ringnut bzw. in die Quernut(en) ein. So greift das Federelement insbesondere in der Mediumszugangsstellung in die eine oder mehreren Quernuten ein. Während der Übergänge von einer Stellung in eine andere wird das Federelement komprimiert.

Das Federelement kann beispielsweise eine Biegefeder oder ein Elastomerring sein, wobei Biegefedern bevorzugt sind. Jedenfalls ist das Federelement geeignet, eine Federkraft auf zwei einander gegenüberliegende Seiten des Konusteils auszuüben, wobei die Federkraft jeweils in Richtung der Längsachse des Konusteils wirkt. Die Biegefeder kann z. B. aus nichtrostendem Federstahl oder aus einem Buntmetall hergestellt sein. Vorzugsweise weist die Biegefeder einen runden Querschnitt auf (Drahtfeder).

Das Federelement ist vorzugsweise in dem Gehäuse befestigt. Die Befestigung ist derart, dass das Federelement nicht ohne weiteres aus dem Gehäuse herausfallen kann, wenn kein Küken in dem Gehäuseinneren angeordnet ist.

In einem zweiten Aspekt betrifft die Erfindung ein medizinisches Endoskop mit mindestens einem Flüssigkeitskanal, dadurch gekennzeichnet, dass es mindestens einen erfindungsgemäßen Absperrhahn umfasst, mittels dem der Flüssigkeitsstrom durch den Flüssigkeitskanal regulierbar ist. 'Regulierbar' bedeutet in diesem Zusammenhang, dass durch die Stellung des Kükens im Absperrhahn geregelt werden kann, ob Flüssigkeit durch den Absperrhahn fließen kann oder nicht. In einer Offenstellung des Absperrhahns kann Flüssigkeit durch den Absperrhahn hindurch fließen, während in einer Geschlossenstellung keine Flüssigkeit durch den Absperrhahn fließen kann. Vorzugsweise kann darüber hinaus auch das Volumen des Flüssigkeitsstroms durch graduelles Schließen des Absperrhahnes reduziert werden.

Das medizinische Endoskop ist vorzugsweise ein gynäkologisches oder urologisches Endoskop. Das Endoskop kann z. B. ein Resektoskop, Hysteroskop, Ureteroskop, Arthroskop oder Zystoskop sein. Alternativ kann das Endoskop auch ein Gastroskop, Koloskop oder Bronchoskop sein. Während erstere üblicherweise mit einem starren Schaftteil versehen sind, umfassen letztere üblicherweise einen flexiblen Schaftteil. Das Endoskop kann als Resektoskop ausgebildet sein.

Das Endoskop weist in typischer endoskopischer Bauweise ein langgestrecktes, rohrartiges Schaftteil auf. Das Schaftteil bzw. Schaftrohr des Endoskops kann z. B. eine Länge von mindestens etwa 150 mm aufweisen, z. B. eine Länge zwischen 150 mm und 400 mm. Der Schaftdurchmesser kann z. B. etwa 3 bis 10 mm betragen. Daraus ergibt sich eine sehr lange und dünne Konfiguration des Schaftrohres. Neben diesem Schaftteil umfasst das Resektoskop zum Halten und Bedienen ein Griffsystem, das üblicherweise aus zwei Griffteilen besteht.

Das Endoskop weist mindestens einen Flüssigkeitskanal auf, d.h. zum Beispiel einen, zwei, drei, vier oder mehr Flüssigkeitskanäle. Ein Flüssigkeitskanal kann durch ein Spülrohr, Innenrohr, Hüllrohr oder dergleichen gebildet werden. Damit das medizinische Fachpersonal während der Behandlung über die Optik eine freie Sicht auf den zu behandelnden Bereich hat, wird während des Eingriffs eine Spülflüssigkeit durch den Flüssigkeitskanal in das Körperinnere hinein geführt oder durch den Flüssigkeitskanal abgeführt. Durch diese Spülflüssigkeit können beispielsweise Gewebestücke, die während einer Resektoskopie freigesetzt werden, weggespült werden. Des Weiteren dient die Spülflüssigkeit dazu, Trübungen, die beispielsweise durch Blut verursacht werden, aus dem Blickfeld des optischen Bildleiters zu entfernen.

Zur Flüssigkeitszufuhr ist ein Flüssigkeitskanal in der Regel im proximalen Bereich des Endoskops einer Spüleinrichtung bzw. einer Pumpe zuordbar, sodass die Spülflüssigkeit zunächst mit einem vorbestimmbaren Druck in das Körperinnere führbar ist. In der Regel wird der Abfluss überschüssiger Flüssigkeit durch einen Flüssigkeitskanal spontan erfolgen. Es ist aber auch möglich, den Abfluss durch Anlegung eines leichten Unterdrucks an den Flüssigkeitskanal sicherzustellen.

Im proximalen Endbereich des Endoskops ist zur Regulierung des Flüssigkeitsstromes im bzw. am Flüssigkeitskanal ferner ein erfindungsgemäßer Absperrhahn angeordnet. Dieser ist vorzugsweise auch im proximalen Endbereich des Flüssigkeitskanals angebracht, zum Beispiel an dessen proximalen Ende. So kann der Absperrhahn beispielsweise geschlossen gehalten werden, wenn noch keine Pumpe bzw. anderweitige Flüssigkeitszufuhr an den Flüssigkeitskanal angeschlossen ist. Der proximale Endbereich kann gegenüber dem Hauptteil des Flüssigkeitskanals abgewinkelt sein. Der Absperrhahn kann beispielsweise als separates Teil ausgebildet sein, das auf beiden Seiten Verbindungselemente zum Anschluss an einen Flüssigkeitskanal bzw. ein Endoskop oder an eine Flüssigkeitszufuhr oder -abfuhr aufweist.

Neben dem oder den Flüssigkeitskanälen kann das Endoskop weitere Komponenten, wie einen optischen Bildleiter, Lichtleitfaserbündel, einen oder mehrere Arbeitskanäle und Durchgangsinstrumente, wie ein Elektrodeninstrument, umfassen.

In noch einem weiteren Aspekt betrifft die Erfindung auch das hierin beschriebene Küken.

### Kurze Beschreibung der Figuren

In den Zeichnungen sind Ausführungsbeispiele der Erfindung schematisch dargestellt. Es zeigen:
- Fig. 1: eine schematische Seitenansicht eines erfindungsgemäßen Endoskops, das einen Absperrhahn aufweist;
- Fig. 2: eine schematische Seitenansicht eines erfindungsgemäßen Absperrhahns;
- Fig. 3: eine schematische Seitenansicht eines erfindungsgemäßen Kükens;
- Fig. 4: eine weitere schematische Seitenansicht des erfindungsgemäßen Kükens aus Fig. 3, wobei das Küken um 90° um die Längsachse (L) seines Konusteils gedreht ist;
- Fig. 5: eine schematische seitliche Schnittdarstellung des erfindungsgemäßen Absperrhahns, wobei sich der Absperrhahn in der Arbeitsstellung und der Offenstellung befindet;
- Fig. 6: eine schematische Schnittdarstellung des erfindungsgemäßen Absperrhahns aus Sicht des Griffteils des Kükens auf Höhe des Nutbodens der ersten Ringnut, wobei sich der Absperrhahn in der Arbeitsstellung befindet;
- Fig. 7: eine schematische seitliche Schnittdarstellung des erfindungsgemäßen Absperrhahns, wobei sich der Absperrhahn in der Mediumszugangsstellung und der Offenstellung befindet;
- Fig. 8: eine schematische Schnittdarstellung des erfindungsgemäßen Absperrhahns aus Sicht des Griffteils des Kükens auf Höhe des Nutbodens der zwei Quernuten, wobei sich der Absperrhahn in der Mediumszugangsstellung befindet;
- Fig. 9: eine schematische Seitenansicht des Endbereichs des Kükens in einer Ausführungsform, die zwei Quernuten aufweist, (links) und eine um 90° gedrehte schematische Seitenansicht dieser Ausführungsform (rechts);
- Fig. 10: eine schematische Seitenansicht des Endbereichs des Kükens in einer Ausführungsform, die zwei Quernuten aufweist, die in eine zweite Ringnut übergehen, (links) und eine um 90° gedrehte schematische Seitenansicht dieser Ausführungsform (rechts);
- Fig. 11: eine schematische Seitenansicht des Endbereichs des Kükens in einer Ausführungsform, die zwei Quernuten und entsprechende Verbindungsnuten aufweist, (links) und eine um 90° gedrehte schematische Seitenansicht dieser Ausführungsform (rechts);
- Fig. 12: eine schematische Schnittdarstellung einer alternativen Ausführungsform des erfindungsgemäßen Absperrhahns aus Sicht des Flüssigkeitskanals, wobei sich der Absperrhahn in der Arbeitsstellung befindet;
- Fig. 13: eine um 90° um die Drehachse des Kükens gedrehte, schematische Schnittdarstellung der Ausführungsform von Fig. 12, wobei sich der Absperrhahn in der Arbeitsstellung befindet; und
- Fig. 14: eine schematische Schnittdarstellung der Ausführungsform von Fig. 12 und 13, aus Sicht des Griffteils des Kükens auf Höhe des Nutbodens der ersten Ringnut, wobei sich der Absperrhahn in der Arbeitsstellung befindet.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

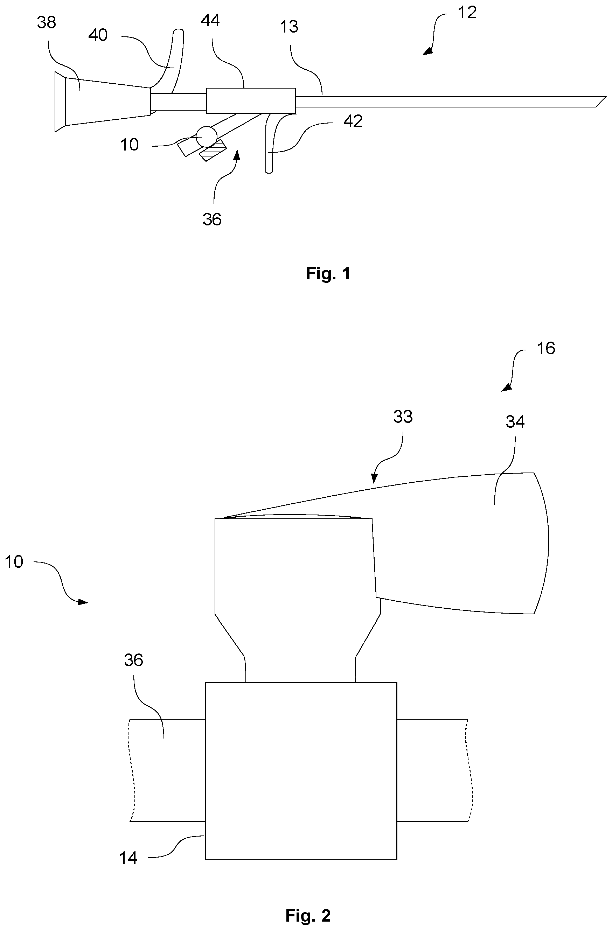

Fig. 1 zeigt eine schematische Seitenansicht eines erfindungsgemäßen Endoskops 12, das einen Absperrhahn 10 aufweist. Das Endoskop 12 weist einen innerhalb des Schaftteils 13 verlaufenden, hier nicht sichtbaren, Flüssigkeitskanal 11 auf, der durch einen Hauptkörper 44 abgewinkelt zu einem Eingangsstutzen 36 führt. An dem Eingangsstutzen 36 ist zur Regulierung des Flüssigkeitsstromes durch den Flüssigkeitskanal 11 ein Absperrhahn 10 angeordnet. Am Eingangsstutzen 36 kann beispielsweise eine Pumpe zur Flüssigkeitszufuhr in den Flüssigkeitskanal 11 angeschlossen sein.

Die hier gezeigte Seitenansicht zeigt das medizinische Endoskop 12 in der Orientierung, in der es üblicherweise während einer Operation verwendet wird. Das Endoskop 12 weist an seinem proximalen Ende ein Okular 38 zur Beobachtung des Operationsgebietes auf. Damit der Operateur das Endoskop 12 während der Operation sicher halten kann, sind zwei Griffstücke 40, 42 an dem Endoskop 12 befestigt. Die Griffstücke 40, 42 sind in üblicher Weise derart ergonomisch geformt, dass der Operateur das Endoskop 12 mit Hilfe der Griffstücke 40, 42 in einer Hand halten kann. Die andere Hand hat der Operateur entsprechend frei, um zum Beispiel Durchgangsinstrumente in einen Arbeitskanal des Instruments einzuführen und zu bedienen.

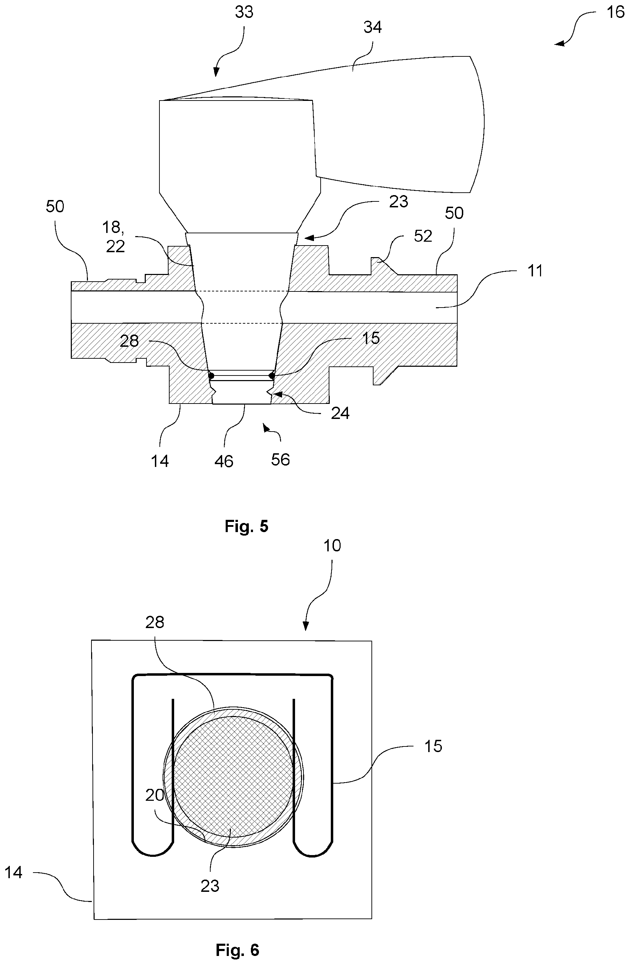

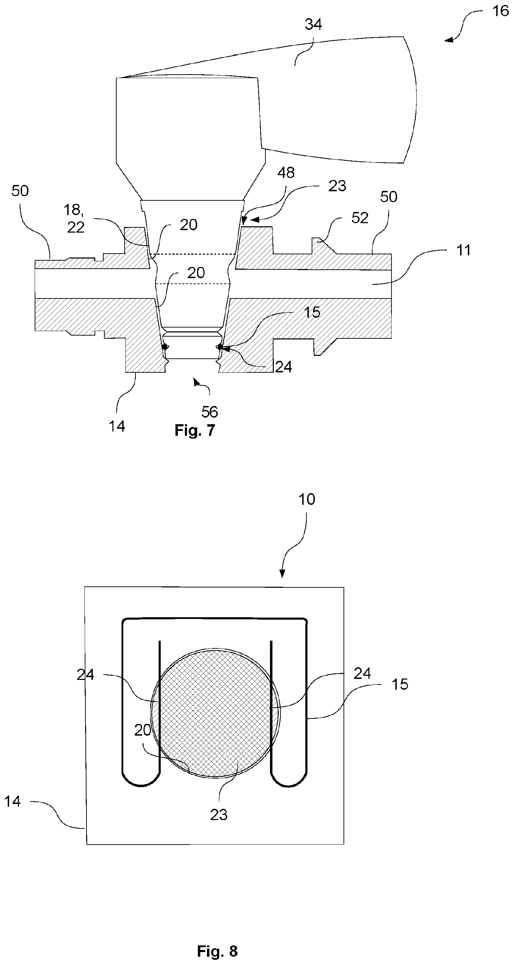

Fig. 2 zeigt eine schematische Seitenansicht eines erfindungsgemäßen Absperrhahns 10, der ein Gehäuse 14 und ein Küken 16 umfasst, das mit seinem hier nicht sichtbaren Konusteil 23 in dem Gehäuse 14 aufgenommen ist. Das Gehäuse 14 ist in etwa quaderförmig und einstückig mit dem rohrförmigen Eingangsstutzen 36 ausgebildet. Alternativ ist es auch denkbar, an beiden Seiten des Gehäuses 14, wie in den Fig. 5 und 7 gezeigt, Verbindungsteile 50 vorzusehen, mittels derer eine Verbindung mit einem Eingangsstutzen 36 hergestellt werden kann. Das Gehäuse 14 weist zur Aufnahme des Kükens 16 einen größeren Umfang und Durchmesser auf, als der Rest des Eingangsstutzens 36.

Wie in Fig. 2 zu sehen, weist das Küken 16 einen Griff 33 auf, der ein Griffteil 34 umfasst, an dem der Benutzer zum Drehen des Kükens 16 anfassen kann. Das Griffteil 34 kann zu diesem Zweck ergonomisch ausgeformt sein, um das Halten und Bewegen durch den Benutzer zu erleichtern.

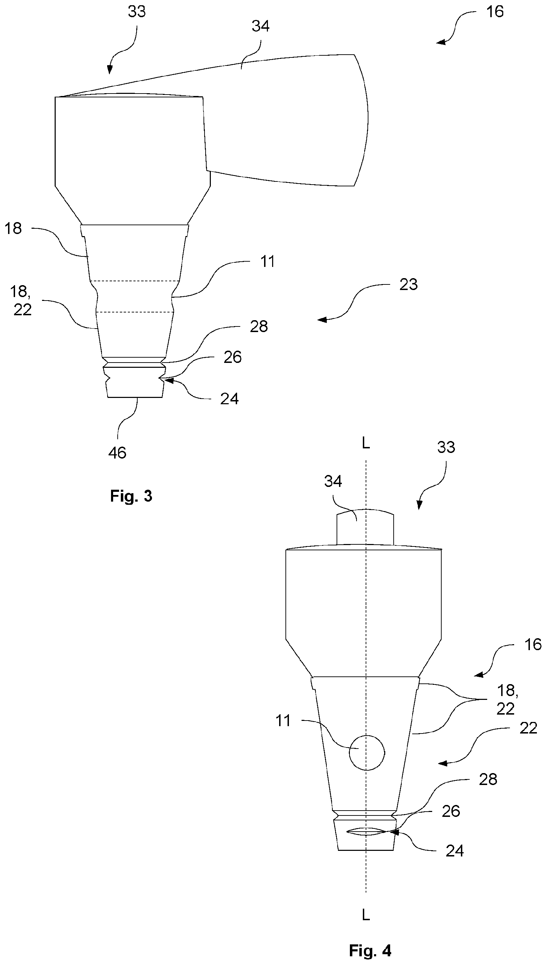

Fig. 3 und Fig. 4 zeigen schematische Seitenansichten eines erfindungsgemäßen Kükens 16, das in seinem Konusteil 23 zwei quer zur Längsachse L des Konusteils 23 verlaufende Quernuten 24 aufweist, deren Nutboden 26 gerade sind. Das Küken 16 ist in der Fig. 4 gegenüber der Fig. 3 um 90° um die Längsachse L seines Konusteils (Drehachse des Kükens) gedreht.

Das Küken 16 besteht zunächst aus einem Konusteil 23, der zur Bildung eines Absperrhahns 10 in ein Gehäuse 14 einführbar ist, und einem Griff 33, der, wie oben beschrieben, zur vereinfachten Bedienung des Absperrhahns 10 ein Griffteil 34 aufweist.

Das Konusteil 23 ist mit einer äußeren Oberfläche ausgebildet, die hierin als Außenwand 22 bezeichnet wird. Die Außenwand 22 umfasst die Außenflächen des Konusteils 23, die radial zur Drehachse L angeordnet sind, d.h. seine Seitenflächen, nicht aber die Stirnfläche 46 des Konusteils 23. Die Außenwand 22 umfasst die Konusfläche 18. Als Konusfläche 18 wird hierin die dichtende Fläche der Außenwand 22 angesehen, die in der Arbeitsstellung dichtend an der Dichtfläche 20 des Gehäuses 14 anliegt.

Das Konusteil 23 wird von einem Flüssigkeitskanal 11 durchspannt, der Form- und Durchmesser-komplementär zu Flüssigkeitskanälen 11 in einem in den Fig. 3 und 4 nicht gezeigten Eingangsstutzen 36, den Verbindungsteilen 50 und dem Gehäuse 14 ist. Die beiden endständigen Öffnungen des im Konusteil 23 angeordneten Flüssigkeitskanals 11 sind jeweils in der Außenwand 22 des Konusteils 23 angeordnet.

Das Konusteil 23 umfasst zwei Quernuten 24, die einander auf der Außenwand 22 des Konusteils 23 gegenüber liegen. Die Quernuten 24 weisen erkennbar jeweils einen geraden Nutboden 26 auf. Sie erstrecken sich somit nicht konzentrisch um die Drehachse des Kükens 16, sondern verlaufen riefenartig an der Seite des Konusteils 23. Die beiden Quernuten 24 weisen dabei einen in etwa trichterförmigen Querschnitt auf.

Das Küken 16 weist ferner eine erste Ringnut 28 auf, die in Längsrichtung auf der Seite der Quernuten 24 angeordnet ist, die dem Griff 33 zugewandt ist bzw. auf der Seite der Quernuten 24, die einen größeren Durchmesser aufweist als die andere Seite der Quernuten 24. Die Nuten (Ringnut 28 und Quernuten 24) sind auf der Seite des Flüssigkeitskanals 11 angeordnet, auf der das Konusteil 23 einen geringeren Durchmesser aufweist. Die Ringnut 28 verläuft erkennbar konzentrisch um die Drehachse des Kükens 16. Die erste Ringnut 28 weist einen in etwa trichterförmigen Querschnitt auf. Die Querschnittsfläche der Ringnut 28 und der Quernuten 24 (für die Quernuten 24 in ihrer Mitte gemessen) ist in der dargestellten Ausführungsform in etwa identisch. Die Querschnittsfläche ist ausreichend, um einen Abschnitt des Federelements 15 aufzunehmen.

Fig. 5 und 7 zeigen schematische seitliche Schnittdarstellungen des erfindungsgemäßen Absperrhahns 10, wobei sich der Absperrhahn in der Arbeitsstellung und der Offenstellung befindet (Fig. 5) bzw. in der Mediumszugangsstellung und der Offenstellung befindet. Die Offenstellung bezeichnet hierin diejenige Stellung, in der (in der Arbeitsstellung) ein maximales Flüssigkeitsvolumen durch den Flüssigkeitskanal 11 des linken Verbindungsteils 50, des Absperrhahns 10 und des rechten Verbindungsteils 50 strömen kann. Der Absperrhahn ist in der Offenstellung somit für den Flüssigkeitsdurchstrom offen. In der in Fig. 5 gezeigten Arbeitsstellung ist das Küken 16 dichtend in das Gehäuse 14 hineingeschoben. Die Konusfläche 18 des Kükens 16 liegt dichtend an der Dichtfläche 20 des Gehäuses 14 an. In der in Fig. 7 gezeigten Mediumszugangsstellung ist das Küken 16 derart aus dem Gehäuse 14 herausgezogen, dass zwischen dem Konusteil 23 und dem Gehäuseinnenraum ein Spalt 48 entsteht, durch den Reinigungs- und/oder Sterilisationsgas strömen kann. Gleichzeitig wird das Küken 16 weiterhin rastend in dem Gehäuse 14 gehalten.

Das Gehäuse 14 weist in Längsrichtung auf beiden Seiten Verbindungsteile 50 auf, die einen Anschluss an einen Eingangsstutzen oder an einen anderen Teil des Endoskops ermöglichen. Hierzu können beispielsweise ein oder beide Verbindungsteile Rastelemente 52 aufweisen, die in den Figuren schematisch dargestellt sind. Das Gehäuse 14 wird, wie auch der Konusteil 23 des Kükens 16, von einem länglichen Flüssigkeitskanal 11 durchlaufen. Darüber hinaus weist das Gehäuse 14 quer zur Längsrichtung des Flüssigkeitskanals 11 eine Ausnehmung 56 auf, die im Wesentlichen eine Konusform aufweist und mindestens abschnittsweise form- und größenkomplementär zu dem Konusteil 23 ist.

Das Küken 16 entspricht dem in Figs. 3 und 4 gezeigten Küken. Es ist mittels eines Federelements 15 sowohl in der Arbeitsstellung (Fig. 5) als auch in der Mediumszugangsstellung (Fig. 7) in dem Gehäuse verrastet. Hierdurch wird verhindert, dass das Küken 16, beispielsweise während der Reinigung aus dem Gehäuse 14 herausfallen kann. In der in Fig. 5 gezeigten Arbeitsstellung greift das Federelement 15 dazu in die erste Ringnut 28 des Kükens 16 ein, in der in Fig. 7 gezeigten Mediumszugangsstellung greift das Federelement 15 aus einander entgegengesetzten Richtungen, d.h. von gegenüberliegenden Seiten, in beide Quernuten 24 ein. Die Federkraft des Federelements 15 wirkt somit in Richtung der Drehachse des Kükens. So wird sichergestellt, dass das Küken 16 nur unter Aufwendung einer größeren Kraft durch Drehen um die Drehachse L von der Offen- in die Geschlossenstellung und *vice* versa bringbar ist und mittels Druckausübung entlang der Längsachse von der Arbeits- in die Mediumszugangsstellung bringbar ist. Das Federelement 15 ist in nicht dargestellter Weise an dem Gehäuse 14 befestigt.

Die Ausbildung und Stellung des Federelements 15 ist in den Fig. 6 und 8 näher erläutert. Die Figuren zeigen schematische Schnittdarstellungen des erfindungsgemäßen Absperrhahns 10 aus Sicht des Griffteils 34 des Kükens 16 auf Höhe des Nutbodens der ersten Ringnut 28, wobei sich der Absperrhahn 10 in der Arbeitsstellung befindet, (Fig. 6) bzw. auf Höhe des Nutbodens der zwei Quernuten 24, wobei sich der Absperrhahn 10 in der Mediumszugangsstellung befindet, (Fig. 8).

Das Federelement 15 ist als Biegefeder mit einem runden Querschnitt, d.h. als metallische Drahtfeder ausgebildet. Das Federelement 15 umschließt dabei den Konusteil 23 klammerartig. Es ist in Fig. 6 erkennbar, dass zwischen dem Federelement 15 und dem Konusteil 23 zwei punktuelle Kontaktflächen bestehen. Hierdurch lässt sich das Küken 16 in der in Fig. 6 gezeigten Arbeitsstellung leichter von der Offenstellung in die Geschlossenstellung drehen, als in der in der Fig. 8 gezeigten Position. Es ist in Fig. 8 erkennbar, dass zwischen dem Federelement 15 und dem Konusteil 23 zwei linienfömige Kontaktflächen bestehen. Die beiden Endbereiche des Federelements 15 greifen hier in die beiden Quernuten 24 ein. Durch die längere Kontaktfläche und den abgewinkelten Übergang zwischen den Enden der Quernuten 24 und der konusförmigen Abschnitte des Kükens 16 lässt sich das Küken 16 in der in Fig. 8 gezeigten Mediumszugangsstellung deutlich schwerer von der Offenstellung in die Geschlossenstellung drehen, als in der in Fig. 6 gezeigten Position.

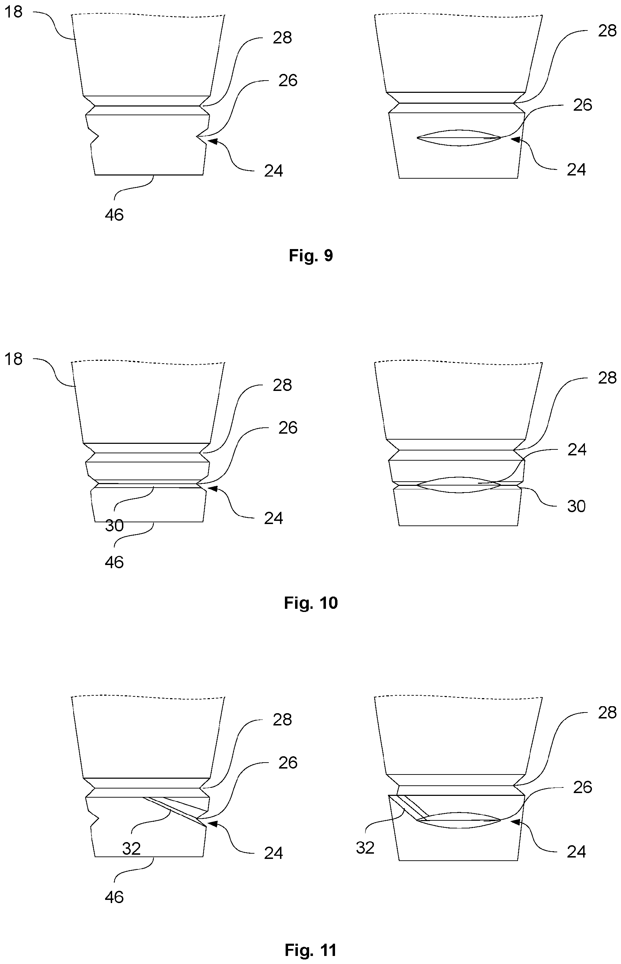

Die Figs. 9 bis 11 zeigen schematische Seitenansichten des Endbereichs des Kükens 16 in drei unterschiedlichen Ausführungsformen. Es ist jeweils auf der linken Seite der Figuren eine Seitenansicht des Endbereichs gezeigt und auf der rechten Seite der Figuren eine um 90° gedrehte schematische Seitenansicht derselben Ausführungsform.

Fig. 9 zeigt eine Ausführungsform, in der das Küken 16 zwei Quernuten 24 aufweist. Diese Ausführungsform entspricht somit der in den Fig. 3 bis 8 dargestellten Ausführungsform. Die Enden der Quernuten 24 gehen jeweils direkt in einen konusförmigen Abschnitt des Kükens 16 über. Die Quernuten 24 sind vollständig von konusförmigen Flächen umgeben.

Im Gegensatz dazu zeigt Fig. 10 eine Ausführungsform, in der die beiden Quernuten 24 jeweils als Abschnitte einer zweiten Ringnut 30 ausgebildet sind, wobei die zweite Ringnut 30 außerhalb der beiden Quernuten 24 parallel zu der ersten Ringnut 28 konzentrisch um die Drehachse des Kükens 16 verläuft. Der Nutboden 26 der Quernuten 24 und der Nutboden der zweiten Ringnut 30 sind äquidistant von der ersten Ringnut 28 beabstandet.

Fig. 11 zeigt eine schematische Seitenansicht des Endbereichs des Kükens 16 in einer Ausführungsform, die zwei Quernuten 24 und zwei Verbindungsnuten 32 aufweist, wobei von den beiden Verbindungsnuten 32 nur eine sichtbar ist. Die Verbindungsnuten 32 verbinden jeweils den in Drehrichtung gelegenen Endbereich der beiden Quernuten 24 mit der ersten Ringnut 28. Die Verbindungsnuten 32 verlaufen dafür schräg zur Längsrichtung und zur Drehrichtung des Konusteils 23. Die Verbindungsnuten 32 wirken bei Drehung des Kükens 16 gewindeartig zusammen. Das Federelement 15 kann auf diese Weise durch Drehung von den Quernuten 24 in die erste Ringnut 28 überführt werden. Gleichzeitig wird der Absperrhahn 10 von der Mediumszugangsstellung in die Arbeitsstellung überführt. Auf diese Weise kann eine Reinigung in der Geschlossenstellung verhindert werden.

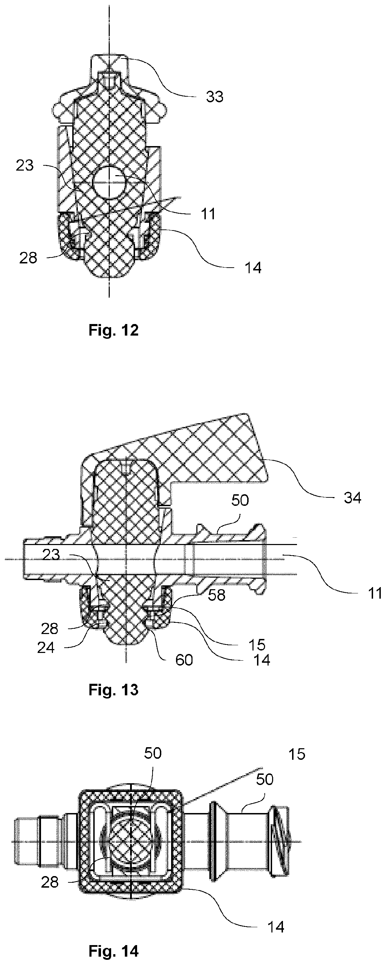

Fig. 12 bis Fig. 14 zeigen schematische Schnittdarstellungen einer alternativen Ausführungsform des erfindungsgemäßen Absperrhahns 10, wobei sich der Absperrhahn 10 in der Arbeitsstellung befindet. Die Schnittdarstellungen sind seitlich aus der Sicht des Flüssigkeitskanals 11 (Fig. 12), eine um 90° um die Drehachse des Konusteils 23 gedrehte Darstellung (Fig. 13) und aus Sicht des Griffteils 34 des Kükens 16 auf Höhe des Nutbodens der ersten Ringnut 28. Die Ausführungsform ist an die in Fig. 9 angelehnt, wobei das Konusteil 23 des Kükens 16 neben der ersten Ringnut 28 und der Quernut 24 zwischen der ersten Ringnut 28 und der Quernut 24 eine erste Ringschulter 58 aufweist, die sich ringförmig um den Umfang des Konusteils 23 erstreckt. Darüber hinaus weist das Konusteil 23 auf der anderen Seite der Quernut 24, der Seite die dem Griffteil 34 abgewandt ist, eine zweite Ringschulter 60 auf. Im Bereich der Ringschultern 58, 60 weist das Konusteil 23 einen größeren Durchmesser auf als in den Bereichen der Nuten. Die zweite Ringschulter 60 sichert das Küken 16 in dem Gehäuse 14.

In der Ausführungsform der Fig. 12 bis 14 ist das Küken 16 zweiteilig ausgebildet, wobei Griff 33 und Konusteil 23 zwei voneinander trennbare Teile sind. Das Küken 16 ist, wie an anderer Stelle beschrieben, mittels eines Federelements 15 in dem Gehäuse 14 gehalten und in der Arbeitsstellung verrastet. Dazu greift das Federelement 15 in die erste Ringnut 28 ein.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

**Bezugszeichenliste**

| | | | |
|---|---|---|---|
| 10 | Absperrhahn | 46 | Stirnfläche |
| 11 | Flüssigkeitskanal | 48 | Spalt |
| 12 | Endoskop | 50 | Verbindungsteil |
| 13 | Schaftteil | 52 | Rastelement |
| 14 | Gehäuse | L | Längsachse Konusteil |
| 15 | Federelement | 56 | Ausnehmung |
| 16 | Küken | 58 | erste Ringschulter |
| 18 | Konusfläche | 60 | zweite Ringschulter |
| 20 | Dichtfläche | | |
| 22 | Außenwand | | |
| 23 | Konusteil | | |
| 24 | Quernut | | |
| 26 | Nutboden | | |
| 28 | erste Ringnut | | |
| 30 | zweite Ringnut | | |
| 32 | Verbindungsnut | | |
| 33 | Griff | | |
| 34 | Griffteil | | |
| 36 | Eingangsstutzen | | |
| 38 | Okular | | |
| 40 | Griffstück | | |
| 42 | Griffstück | | |
| 44 | Hauptkörper | | |

## Patentansprüche

1. Absperrhahn (10) für einen Flüssigkeitskanal (11) eines medizinischen Endoskops (12), mit einem Gehäuse (14), einem Federelement (15) und einem drehbar in dem Gehäuse (14) gelagerten Küken (16), wobei das Küken (16) in einer Arbeitsstellung mit einer Konusfläche (18) an einer Dichtfläche (20) des Gehäuses (14) anliegt und in eine Mediumszugangsstellung bringbar ist, in der sich die Konusfläche (18) und die Dichtfläche (20) außer Anlage befinden und das Gehäuse (14) und das Küken (16) verbunden bleiben, wobei das Küken (16) in der Außenwand (22) eines Konusteils (23) eine oder mehrere quer zur Längsachse des Konusteils (23) verlaufende Quernuten (24) aufweist, deren Nutböden (26) gerade sind, und das Federelement (15) in der Mediumszugangsstellung in die eine oder mehreren Quernuten (24) eingreift.

2. Absperrhahn (10) nach Anspruch 1, **dadurch gekennzeichnet, dass** das Küken (16) parallel zur Quernut (24) eine erste konzentrisch verlaufende Ringnut (28) aufweist und das Federelement (15) in der Arbeitsstellung in die erste Ringnut (28) eingreift.

3. Absperrhahn (10) nach Anspruch 2, **dadurch gekennzeichnet, dass** das Küken (16) zwei Quernuten (24) aufweist, die sich vorzugsweise auf dem Konusteil (23) gegenüber liegen.

4. Absperrhahn (10) nach einem der vorangehenden Ansprüche, **dadurch gekennzeichnet, dass** die eine oder mehrere Quernuten (24) jeweils als Abschnitt einer zweiten Ringnut (30) ausgebildet sind, wobei die zweite Ringnut (30) außerhalb der einen oder mehreren Quernuten (24) parallel zu der ersten Ringnut (28) konzentrisch verläuft.

5. Absperrhahn (10) nach einem der vorangehenden Ansprüche, **dadurch gekennzeichnet, dass** das Küken (16) in der Außenwand (22) seines Konusteils (23) eine oder mehrere Verbindungsnuten (32) aufweist, die jeweils ein Ende einer Quernut (24) mit der ersten Ringnut (28) verbinden.

6. Absperrhahn (10) nach Anspruch 5, **dadurch gekennzeichnet, dass** die eine oder mehrere Verbindungsnuten (32) schräg zur Längsachse der ersten Ringnut (28) angeordnet sind.

7. Absperrhahn (10) nach einem der vorangehenden Ansprüche, **dadurch gekennzeichnet, dass** die Quernut (24) einen trapezförmigen Querschnitt aufweist.

8. Absperrhahn (10) nach einem der vorangehenden Ansprüche, **dadurch gekennzeichnet, dass** das Federelement (15) eine Biegefeder oder ein Elastomerring ist.

9. Absperrhahn (10) nach Anspruch 8, **dadurch gekennzeichnet, dass** die Biegefeder einen runden Querschnitt aufweist.

10. Medizinisches Endoskop (12) mit mindestens einem Flüssigkeitskanal (11), **dadurch gekennzeichnet, dass** es mindestens einen Absperrhahn (10) nach einem der vorangehenden Ansprüche umfasst, mittels dem der Flüssigkeitsstrom durch den Flüssigkeitskanal (11) regulierbar ist.

## Claims

1. A stopcock (10) for a liquid channel (11) of a medical endoscope (12), having a housing (14), a spring element (15) and a plug (16) rotatably mounted in the housing (14), wherein the plug (16) in a working position lies with a conical surface (18) against a seal surface (20) of the housing (14) and can be placed in a medium access position in which the conical surface (18) and the seal surface (20) have no contact and the housing (14) and the plug (16) remain connected, wherein the plug (16) comprises one or more transverse grooves (24), in the outer wall (22) of a conical piece (23), running transversely to the longitudinal axis of the conical piece (23) and having straight groove bottoms (26), and the spring element (15) in the medium access position engages with the one or more transverse grooves (24).

2. The stopcock (10) according to claim 1, **characterized in that** the plug (16) comprises a first concentrically running annular groove (28) parallel to the transverse groove (24) and the spring element (15) in the working position engages with the first annular groove (28).

3. The stopcock (10) according to claim 2, **characterized in that** the plug (16) comprises two transverse grooves (24), preferably situated opposite each other on the conical piece (23).

4. The stopcock (10) according to any one of the preceding claims, **characterized in that** the one or more transverse grooves (24) are each configured as a segment of a second annular groove (30), wherein the second annular groove (30) runs concentrically parallel to the first annular groove (28) outside of the one or more transverse grooves (24).

5. The stopcock (10) according to any one of the preceding claims, **characterized in that** the plug (16) comprises one or more connection grooves (32) in the outer wall (22) of its conical piece (23), each of them joining one end of a transverse groove (24) to the first annular groove (28).

6. The stopcock (10) according to claim 5, **characterized in that** the one or more connection grooves (32) are arranged at a slant to the longitudinal axis of the first annular groove (28).

7. The stopcock (10) according to any one of the preceding claims, **characterized in that** the transverse groove (24) has a trapezoidal cross section.

8. The stopcock (10) according to any one of the preceding claims, **characterized in that** the spring element (15) is a bending spring or an elastomer ring.

9. The stopcock (10) according to claim 8, **characterized in that** the bending spring has a round cross section.

10. A medical endoscope (12) having at least one liquid channel (11), **characterized in that** it comprises at least one stopcock (10) according to any one of the preceding claims, by means of which the liquid flow through the liquid channel (11) can be regulated.

## Revendications

1. Robinet d'arrêt (10) pour un canal de liquide (11) d'un endoscope médical (12), avec un boîtier (14), un élément à ressort (15) et un coude (16) monté de manière rotative dans le boîtier (14), le coude (16), dans une position de travail, s'appliquant par une surface conique (18) sur une surface d'étanchéité (20) du boîtier (14) et pouvant être amené dans une position d'accès au fluide, dans laquelle la surface conique (18) et la surface d'étanchéité (20) se trouvent hors de contact et le boîtier (14) et le coude (16) restent reliés, le coude (16) présentant dans la paroi extérieure (22) d'une partie conique (23) une ou plusieurs rainures transversales (24) s'étendant transversalement à l'axe longitudinal de la partie conique (23), dont les fonds de rainure (26) sont droits, et l'élément à ressort (15) s'engageant dans les une ou plusieurs rainures transversales (24) dans la position d'accès au fluide.

2. Robinet d'arrêt (10) selon la revendication 1, **caractérisé en ce que** le coude (16) présente une première rainure annulaire s'étendant de manière concentrique (28) parallèlement à la rainure transversale (24) et l'élément à ressort (15) s'engage dans la première rainure annulaire (28) dans la position de travail.

3. Robinet d'arrêt (10) selon la revendication 2, **caractérisé en ce que** le coude (16) présente deux rainures transversales (24), qui se trouvent de préférence en face l'une de l'autre sur la partie conique (23) .

4. Robinet d'arrêt (10) selon l'une quelconque des revendications précédentes, **caractérisé en ce que** les une ou plusieurs rainures transversales (24) sont chacune réalisées sous forme de section d'une deuxième rainure annulaire (30), la deuxième rainure annulaire (30) s'étendant en dehors des une ou plusieurs rainures transversales (24) parallèlement à la première rainure annulaire (28) de manière concentrique.

5. Robinet d'arrêt (10) selon l'une quelconque des revendications précédentes, **caractérisé en ce que** le coude (16) présente, dans la paroi extérieure (22) de sa partie conique (23), une ou plusieurs rainures de liaison (32) qui relient chacune une extrémité d'une rainure transversale (24) à la première rainure annulaire (28).

6. Robinet d'arrêt (10) selon la revendication 5, **caractérisé en ce que** les une ou plusieurs rainures de liaison (32) sont agencées en oblique par rapport à l'axe longitudinal de la première rainure annulaire (28).

7. Robinet d'arrêt (10) selon l'une quelconque des revendications précédentes, **caractérisé en ce que** la rainure transversale (24) présente une section transversale trapézoïdale.

8. Robinet d'arrêt (10) selon l'une quelconque des revendications précédentes, **caractérisé en ce que** l'élément à ressort (15) est un ressort de flexion ou un anneau en élastomère.

9. Robinet d'arrêt (10) selon la revendication 8, **caractérisé en ce que** le ressort de flexion présente une section transversale ronde.

10. Endoscope médical (12) avec au moins un canal de liquide (11), **caractérisé en ce qu'**il comprend au moins un robinet d'arrêt (10) selon l'une quelconque des revendications précédentes, au moyen duquel le flux de liquide à travers le canal de liquide (11) peut être régulé.
